# EUROPÄISCHE PATENTSCHRIFT

(11) **EP 1 889 070 B1**
(45) Veröffentlichungstag und Bekanntmachung des Hinweises auf die Patenterteilung: **24.06.2009**
(21) Anmeldenummer: 06742337.6
(22) Anmeldetag: 13.05.2006
(51) Int. Cl.: G01N 33/68

(54) **VERFAHREN ZUR BESTIMMUNG DER KONZENTRATION VON ASYMMETRISCHEM DIMETHYLARGININ (ADMA)**
METHOD FOR DETERMINING THE CONCENTRATION OF ASYMMETRIC DIMETHYLARGININE (ADMA)
PROCEDE POUR DETERMINER LA CONCENTRATION DE DIMETHYLARGININE ASYMETRIQUE (ADMA)

(30) Priorität: 28.05.2005 DE 102005024531; 26.11.2005 DE 102005056408
(43) Veröffentlichungstag der Anmeldung: 20.02.2008
(73) Patentinhaber: Medizinische Fakultät, 39120 Magdeburg (DE); ESA Patentverwertungsagentur Sachsen-Anhalt GmbH, 39114 Magdeburg (DE)
(72) Erfinder: BODE-BÖGER, Stefanie, M., 30880 Laatzen (DE); MARTENS-LOBENHOFFER, Jens, 22339 Hamburg (DE)
(74) Vertreter: Fischer, Volker
(86) Internationale Anmeldenummer: PCT/DE2006/000829
(87) Internationale Veröffentlichungsnummer: WO 2006/128419

(56) Entgegenhaltungen:
- KIRCHHERR HARTMUT ET AL: "HPLC-tandem mass spectrometric method for rapid quantification of dimethylarginines in human plasma." CLINICAL CHEMISTRY. JAN 2005, Bd. 51, Nr. 1, Januar 2005 (2005-01), Seiten 249-252, XP002399825 ISSN: 0009-9147
- SHOU ET AL: "Simple means to alleviate sensitivity loss by trifluoroacetic acid (TFA) mobile phases in the hydrophilic interaction chromatography-electrospray tandem mass spectrometric (HILIC-ESI/MS/MS) bioanalysis of basic compounds" JOURNAL OF CHROMATOGRAPHY B: BIOMEDICAL SCIENCES & APPLICATIONS, ELSEVIER, AMSTERDAM, NL, Bd. 825, Nr. 2, 5. Februar 2005 (2005-02-05), Seiten 186-192, XP005102442 online ISSN: 1570-0232 Gefunden im Internet: URL:http://www.sciencedirect.com/science?_ ob=ArticleURL&_udi=B6X0P-4FDMY92-2&_user=9 87766&_coverDate=10%2F25%2F2005&_alid=4532 84165&_rdoc=1&_fmt=summary&_orig=search&_c di=7220&_sort=d&_st=0&_docanchor=&_acct=C0 00049880&_version=1&_urlVersion=0&_userid= 987766&md5=fa318f48ca153f63dbc22ff2c3310d8 c>
- GEHRIG P M ET AL: "Fragmentation pathways of N<G>-methylated and unmodified arginine residues in peptides studied by ESI-MS/MS and MALDI-MS" JOURNAL OF THE AMERICAN SOCIETY FOR MASS SPECTROMETRY, ELSEVIER SCIENCE INC, US, Bd. 15, Nr. 2, 2004, Seiten 142-149, XP004488227 ISSN: 1044-0305
- VISHWANATHAN K ET AL: "Determination of arginine and methylated arginines in human plasma by liquid chromatography-tandem mass spectrometry" JOURNAL OF CHROMATOGRAPHY. BIOMEDICAL APPLICATIONS, ELSEVIER, AMSTERDAM, NL, Bd. 748, Nr. 1, 1. Oktober 2000 (2000-10-01), Seiten 157-166, XP004224393 ISSN: 0378-4347
- MARTENS-LOBENHOFFER JENS ET AL: "Determination of arginine and asymmetric dimethylarginine (ADMA) in human plasma by liquid chromatography/mass spectrometry with the isotope dilution technique." JOURNAL OF MASS SPECTROMETRY : JMS. NOV 2004, Bd. 39, Nr. 11, November 2004 (2004-11), Seiten 1287-1294, XP002399826 ISSN: 1076-5174
- SCHWEDHELM EDZARD: "Quantification of ADMA: analytical approaches." VASCULAR MEDICINE (LONDON, ENGLAND) JUL 2005, Bd. 10 Suppl 1, 1. Mai 2005 (2005-05-01), Seiten S89-S95, XP009072520 Internet ISSN: 1358-863X Gefunden im Internet: URL:http://vmj.sagepub.com/content/vol10/2 _suppl/>
- MARTENS-LOBENHOFFER JENS ET AL: "Fast and efficient determination of arginine, symmetric dimethylarginine, and asymmetric dimethylarginine in biological fluids by hydrophilic-interaction liquid chromatography-electrospray tandem mass spectrometry." CLINICAL CHEMISTRY. MAR 2006, Bd. 52, Nr. 3, März 2006 (2006-03), Seiten 488-493, XP001247520 ISSN: 0009-9147

## Beschreibung

Die Erfindung betrifft ein Verfahren zur Bestimmung der Konzentration von asymmetrischem Dimethylarginin (ADMA) simultan mit Arginin und symmetrischem Dimethylarginin (SDMA) in biologischen Proben mittels HPLC-MS-MS.

Die Aminosäure Arginin dient in biologischen Systemen unter anderem als Ausgangsstoff für die Synthese des Botenstoffes Stickstoffmonoxid (NO) durch die Enzyme der Gruppe der Stickstoffmonoxygenasen (NOS). Die ausreichende Verfügbarkeit dieses Botenstoffs ist unter anderem Voraussetzung für die physiologische Funktion der Endothelzellen im kardiovaskulären System, andererseits ist ein Mangel an NO mit endothelialer Dysfunktion und den damit einhergehenden Krankheitsbildern wie Arteriosklerose, Bluthochdruck oder Schlaganfall verbunden.
In Proteine integriertes Arginin wird im Körper auf einem weiteren Stoffwechselweg über die Enzymgruppe der Protein-Methyl-Transferasen (PRMT) zu in Proteine integriertem Monomethylarginin (MMA), asymmetrischem Dimethylarginin (ADMA) und symmetrischem Dimethylarginin (SDMA) umgesetzt. Beim enzymatischen Proteinabbau werden diese Aminosäuren freigesetzt. Die so freigesetzten methylierten Argininderivate MMA und ADMA hemmen die Funktion von NOS und vermindern auf diese Weise die Produktion von NO. MMA spielt dabei nur eine untergeordnete Rolle, da seine Wirkkonzentration nur etwa 10% derjenigen von ADMA erreicht. SDMA selbst hemmt NOS nicht, teilt aber mitADMA den Transportmechanismus durch die Zellmembranen und kann sich so indirekt auf die Produktionsrate von NO auswirken.
Erhöhte Plasmaspiegel von ADMA sind in einer Reihe von Krankheitsbildern wie Bluthochdruck, Hypercholesterinämie, Nierenfunktionsstörungen oder Diabetes Mellitus beschrieben und es ist mit hoher Sicherheit anzunehmen, dass ein erhöhter Plasmaspiegel von ADMA ein direkter Risikofaktor für kardiovaskuläre Erkrankungen ist.
Zur Erforschung derartiger Krankheiten und von Therapieansätzen ist ein ständig steigender Bedarf an Bestimmungen von ADMA in verschiedenen biologischen Flüssigkeiten wie Blutplasma, Urin oder Zellkulturmedium zu erwarten.
ADMA wird in biologischen Systemen sowohl über die Niere in unveränderter Form ausgeschieden oder enzymatisch zu Citrullin und Dimethylamin aufgespalten. Dies bedeutet, dass ADMA und SDMA bei Patienten mit Niereninsuffizienz im Blut ansteigt. Eine Nierentransplantation normalisiert die SDMA-Spiegel, aber senkt die ADMA-Spiegel nur im geringem Ausmaß. Das liegt daran, dass der Hauptmetabolisierungsweg speziell für ADMA das Enzym Dimethylarginin-Dimethylaminohydrolase (DDAH) ist (SDMA wird nicht abgebaut). Es existieren 2 Isoformen: die DDAH I ist in Geweben mit neuronaler NOS gefunden worden, die DDAH II findet sich in Geweben mit endothellaler NOS. Eine Vielzahl pathologischer Stimuli erhöhen oxidativen Stress wie z.B. oxidiertes LDL-Cholesterin, Zytokine, Hyperhomozysteinämie oder Hyperglykämie. Jeder dieser Stimuli führt durch gesteigerte Sauerstoffradikalbildung zu einer Abschwächung der DDAH Aktivität und damit zu einer Erhöhung der ADMA- Konzentration, wie dies in der Figur zum Stand der Technik veranschaulicht ist. Durch Gabe von Antioxidantien kann in vitro dieser Effekt wieder aufgehoben werden und die DDAH- Aktivität wieder hergestellt werden.
Die Aktivität von DDAH ist von entscheidender Bedeutung für die Gleichgewichtskonzentration von ADMA in biologischen Systemen. Es ist bekannt, dass die Aktivität von DDAH von externen Faktoren wie oxidativer Stress beeinflusst wird. Eine verringerte Aktivität führt zu erhöhten ADMA Spiegeln mit ihren negativen Konsequenzen.
Die quantitative Bestimmung von ADMA in biologischen Proben wird durch die Tatsache erschwert, dass neben ADMA in solchen Proben immer auch eine sehr große Zahl von anderen Konstituenten vorhanden ist, wobei die anderen physiologischen Aminosäuren und insbesondere das zu ADMA strukturähnliche SDMA eine Quantifizierung erschweren. Aus der Literatur bekannt sind eine Reihe von Methoden zur Bestimmung von ADMA in biologischen Proben. Am weitesten verbreitet ist die Methodik der Hochdruck-Flüssigkeischromatographie mit Fluoreszensdetektion. Dazu werden Arginin, ADMA, SDMA gegebenenfalls MMA und ein interner Standard aus der biologischen Probe mittels lonenaustausch-Festphasenextraktion (SPE) extrahiert, der Extrakt wird mit Orthophthalaldehyd und einem Mercaptan (z.B. 2-Mercaptoethanol, 3-Mercaptopropionsäure) zu einem fluoreszierenden Derivat umgesetzt, die Derivate der genannten Verbindungen werden mittels HPLC aufgetrennt und mittels Fluoreszenzdetektion quantitativ bestimmt. Eine große Zahl von Modifikationen dieser Methodik sind beschrieben, wobei das Prinzip allerdings stets gleich bleibt. (siehe z. B. Chen BM, Xia LW, Zhao RQ. Determination of N(G),N(G)-dimethylarginine in human plasma by high-performance liquid chromatography. J Chromatogr B Biomed Sci Appl 1997; 692: 467-71, Meyer J, Richter N, Hecker M. High-performance liquid chromatographic determination ofnitric oxide synthase-related arginine derivatives in vitro and in vivo. Anal Biochem 1997; 247: II-6,
Pettersson A, Uggla L, Backman V Determination of dimethylated arginines in human plasma by high- performance liquid chromatography. J Chromatogr B Biomed Sci Appl 1997; 692: 257-62,
Pi J, Kumagai Y, Sun G, Shimojo N. Improved method for simultaneous determination of L-arginine and its mono- and dimethylated metabolites in biological samples by high-performance liquid chromatography. J Chromatogr B Biomed Sci Appl 2000; 742: 199-203,
Dobashi Y, Santa T, Nakagomi K, Imai K. An automated analyzer for methylated arginines in rat plasma by high-pertormance liquid chromatography with post-column fluorescence reaction. Analyst 2002; 127: 54-9,
Teerlink T, Nijveldt RJ, de Jong S, van Leeuwen PAM. Determination of arginine, asymmetric dimethylarginine, and symmetric dimethylarginine in human plasma and other biological samples by high-performance liquid chromatography. Anal Biochem 2002; 303: 131-7,
Marra M, Bonfigli AR, Testa R, Testa I, Gambini A, Coppa G. High-performance liquid chromatographic assay of asymmetric dimethylarginine, symmetric dimethylarginine, and arginine in human plasma by derivatization with naphthalene-2,3-dicarboxaldehyde. Anal Biochem 2003; 318: 13-7,
Heresztyn T, Worthley MI, Horowitz JD. Determination of I-arginine and N(G),N(G)- and N(G),N(G')-dimethyl-I-arginlne in plasma by liquid chromatography as AccQ-Fluor trade mark fluorescent derivatives. J Chromatogr B Analyt Technol Biomed Life Sci 2004; 805: 325-9 und
Zhang WZ, Kaye DM. Simultaneous determination of arginine and seven metabolites in plasma by reversed-phase liquid chromatography with a time-controlled ortho-phthaldialdehyde precolumn derivatization. Anal Biochem 2004; 326: 87-92). Hauptnachteil dieser Methodik ist die Extraktion mittels SPE, die arbeitsintensiv, teuer und fehlerträchtig ist. Weitere chromatographische Trennverfahren beruhen aufKapillarelektrophorese mit Fluoreszenzdetektion (siehe z. B. Causse E, Siri N, Arnal JF, Bayle C, Malatray P, Valdiguie P et al. Determination of asymmetrical dimethylarginine by capillary electrophoresis-laserinduced fluorescence. J Chromatogr B Biomed Sci Appl 2000; 741: 77-83 und Trapp G, Sydow K, Dulay MT, Chou T, Cooke JP, Zare RN. Capillary electrophoretic and micellar electrokinetic separations of asymmetric dimethyl-L-arginine and structurally related amino acids: quantitation in human plasma J Sep Sci 2004; 27: 1483-90) oder Gaschromatographie mit massenspektrometrischer Detektion nach jeweils entsprechender Extraktion und Derivatisierung von ADMA und gegebenenfalls Arginin und SDMA (siehe z. B.Tsikas D, Schubert B, Gutzki FM, Sandmann J, Frolich JC. Quantitative determination of circulating and urinary asymmetric dimethylarginine (ADMA) in humans by gas chromatography-tandem mass spectrometry as methyl ester tri(N-pentafluoropropionyl) derivative. J Chromatogr B Analyt Technol Biomed Life Sci 2003; 798: 87-99 und Albsmeier J, Schwedhelm E, Schulze F, Kastner M, Boger RH. Determination ofN(G),N(G)-dimethyl-I-arginine, an endogenous NO synthase inhibitor, by gas chromatography-mass spectrometry. J Chromatogr B Analyt Technol Biomed Life Sci 2004; 809: 59-65). Neuere Verfahren, die die HPLC-MS Technologie verwenden, sind ebenfalls beschrieben worden (siehe Huang LF, Guo FQ, Liang YZ, Li BY, Cheng BM (2004) Simultaneous determination of L-arginine and its mono- and dimethylated metabolites in human plasma by high-performance liquid chromatography-mass spectrometry. Anal Bioanal Chem 380: 643-649,
Martens-Lobenhoffer J, Krug O, Bode-Boger SM (2004) Determination of arginine and asymmetric dimethylarginine (ADMA) in human plasma by liquid chromatography/mass spectrometry with the isotope dilution technique. J Mass Spectrom 39:1287-1294, Kirchherr H, Kuhn-Velten WN (2005) HPLC-tandem mass spectrometric method for rapid quantification of dimethylarginines in human plasma. Clin Chem 51:249-252, Schwedhelm E, Tan-Andresen J, Maas R, Riederer U, Schulze F, Boger RH (2005) Liquid chromatography-tandem mass spectrometry method for the analysis of asymmetric dimethylarginine in human plasma. Clin Chem 51:1268-1271). GEHRIG P M ET AL: "Fragmentation pathways of N<G>-methylated and unmodified arginine residues in peptides studied by ESI-MS/MS and MALDI-MS" JOURNAL OF THE AMERICAN SOCIETY FOR MASS SPECTROMETRY, Bd. 15, Nr. 2, 2004, Seiten 142-149. VISHWANATHAN K ET AL: "Determination of arginine and methylated arginines in human plasma by liquid chromatography-tandem mass spectrometry" JOURNAL OF CHROMATOGRAPHY. BIOMEDICAL APPLICATIONS, Bd. 748, Nr. 1, 1. Oktober 2000 (2000-10-01), Seiten 157-166.SCHWEDHELM EDZARD: "Quantification of ADMA: analytical approaches." VASCULAR MEDICINE (LONDON, ENGLAND) JUL 2005, Bd. 10 Suppl 1, 2005-05-01, Seiten S89-S95. Diese Verfahren benötigen teils eine Derivatisierung der Analyten vor der Chromatographie oder müssen ADMA und SDMA vollständig chromatographisch trennen, um zuverlässige quantitative Ergebnisse zu erhalten. Ein prinzipiell anderer Ansatz zur Bestimmung von ADMA in biologischen Proben stellt die Bestimmung mittels immunologischer Reaktionen mit spezifischen Antikörpern dar. Kommerziell erhältliche Reagenzienkits arbeiten nach dem ELISA (enzyme-linked immunosorbent assay) Verfahren. Prinzipiell kann mit diesem Verfahren immer nur ein einziger Analyt, hier also ADMA, quantifiziert werden. Die gleichzeitige Bestimmung von z.B. Arginin oder SDMA, wie bei den chromatographischen Verfahren möglich, ist ausgeschlossen.

Das Ziel der Erfindung besteht in der Beseitigung der Nachteile bei den bekannten Verfahren zur Bestimmung der Konzentration von asymmetrischem Dimethylarginin (ADMA) simultan mit Arginin und symmetrischem Dimethylarginin (SDMA) in biologischen Proben mittels HPLC-MS-MS. Dazu besteht die Aufgabe, ein preisgünstiges Verfahren zu entwickeln, das die routinemäßige Aufarbeitung großer Probenmengen mit präzisen Aussagen gestattet.

Erfindungsgemäß wird diese Aufgabe durch das Verfahren nach Anspruch 1 gelöst.

Ein entscheidender Fortschritt des hier beschriebenen Verfahrens zur Messung von Arginin, ADMA und SDMA besteht in der minimalen Probenvorbereitung. Eine arbeitsintensive, teure und wenig zuverlässige Probenextraktion mittels SPE entfällt somit völlig, ebenso wie eine Derivatisierung der Analyten vor der chromatographischen Trennung. Eine weitere Innovation ist die chromatographische Abtrennung der Analyten von den Matrixbestandteilen auf einer sehr preisgünstigen Normalphasentrennsäule (Silica) mit einem Wasser / Acetonitril / Propionsäure / Trifluoressigsäure 10 / 90 / I / 0,025 Gemisch als mobiler Phase. Unter diesen Bedingungen lassen sich die extrem polaren Substanzen schnell und ohne vorherige Derivatisierung auftrennen, während auf konventionellen Umkehrphasen-Trennsäulen praktisch keine Retention und damit Abtrennung von Matrixbestandteilen zu erreichen ist. Eine vollständige chromatographische Trennung von ADMA und SDMA ist nicht mehr notwendig, da durch die Verwendung eines Tandem-Massenspektrometers als DetektorADMA und SDMA vollständig über ihre MS-MS-Spektren unterschieden werden können. Die Massenspektrometrische Detektion hat den weiteren Vorteil gegenüber den bekannten Methoden mit Fluoreszensdetektion, dass aufgrund der extrem hohen Selektivität Interferenzen und damit Quantifizierungsfehler praktisch ausgeschlossen werden können. Die Verwendung von isotopenmarkierten internen Standards führt zu einer besonders hohen Präzision und Richtigkeit bei der Quantifizierung, unabhängig von der Probenmatrix. Unkontrollierbare systematische Fehler, die bei der Messung von biologischen Proben mit konventionellen Methoden auftreten können, entfallen hier völlig. Dies gilt ebenfalls für die Quantifizierung von ADMA mit ELISA-Kits. Der einzige kommerziell erhältliche ADMA-EUSA-Kit (DLD-Diagnostika GmbH, Deutschland) muss für jede Probenmatrix (z.B. Blutplasma, Blutserum, Zellkulturmedium) einzeln kalibriert werden. Die Ergebnisse sind darüber hinaus, wie unsere eigenen Kontrollmessungen ergeben haben, abhängig vom jeweiligen Krankheitstyp bei ansonsten gleicher Probenmatrix. Des weiteren ist ein ELISA-Verfahren prinzipiell nur in der Lage, einen einzelnen Analyten zu bestimmen. Arginin und SDMA müssen mit einem anderen Verfahren ebenfalls quantifiziert werden, um unter Verwendung weiterer klinischer Parameter wie z.B. Endothelfunktion ein aussagefähiges Gesamtbild zum NO-Status eines biologischen Systems erstellen zu können. Das vorgeschlagene Analyseverfahren bietet die Möglichkeit Arginin, ADMA sowie SDMA simultan zu bestimmen.

Zur Erforschung von kardiovaskulären Krankheiten ist die Bestimmung von Arginin, ADMA und SDMA im Blutplasma und Urin von Patienten und in Zellkulturmedien notwendig. Die Zahl der privaten biochemischen Laboratorien, die eine derartige Analytik routinemäßig anbieten, wächst stetig. Das Interesse an preiswerten, wenig arbeitsintensiven und präzisen Analyseverfahren ist daher groß. Das hier beschriebene erfindungsgemäße Verfähren erfüllt diese Anforderungen.

Die Erfindung soll nachfolgend anhand eines Ausführungsbeispieles näher erläutert werden. Die zugehörigen Abbildungen zeigen in
- Figur 1:: ein typisches Chromatogramm für Blutplasma und in
- Figur 2:: Blutplasmawerte für Arginin, ADMA und SDMA von Normalprobanden und dialysepflichtigen Patienten.

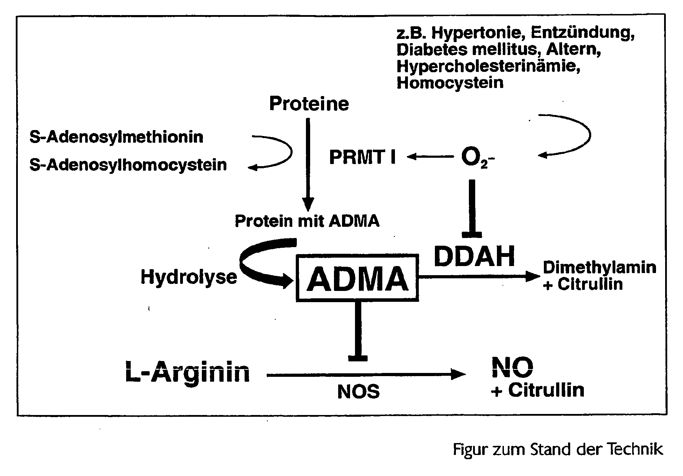

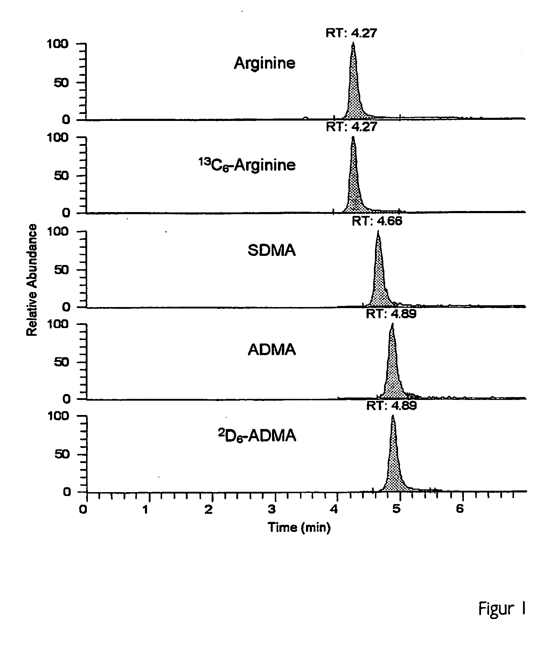

Die Probenvorbereitung für die quantitative Analyse von Arginin, ADMA und SDMA beinhaltet nur zwei schnell und einfach ausführbare Schritte. Als erster Schritt wird die Probe (Blutplasma, Urin oder Zellkulturmedium) mit einer Lösung der isotopenmarkierten internen Standards ¹³C₆-Arginin (käuflich) und D₆-ADMA (hergestellt nach Martens-Lobenhoffer J, Krug O, Bode-Boger SM (2004) Determination of arginine and asymmetric dimethylarginine (ADMA) in human plasma by liquid chromatography/mass spectrometry with the isotope dilution technique. J Mass Spectrom 39:1287-1294) versetzt. Im zweiten Schritt werden zu einem Teil dieser Probe 9 Teile eines Gemisches aus Acetonitril / Propionsäure / Trifluoressigsäure 99 / 1 / 0,025 (Volumenanteile) gegeben. Hierbei werden die in der Probe vorhandenen Proteine ausgefällt und können abzentrifugiert werden. Des weiteren wird dadurch die Zusammensetzung der Probe derjenigen des Laufmittels (siehe unten) für die HPLC-Trennung angenähert, so dass die chromatographische Trennung nicht durch die Injektion von Proben mit einer von der mobilen Phase unterschiedlichen Elutionskraft beeinträchtigt wird. Die so vorbereiteten Proben werden direkt der HPLC-Trennung zugeführt Diese erfolgt auf einer Silica Normalphasensäule, wobei als mobile Phase kein typisches Normalphasenlaufmittel (wie z.B. Hexan oder Dichlormethan) verwendet wird, sondern aus einem Gemisch aus Wasser / Acetonitril / Propionsäure / Trifluoressigsäure besteht. Das Volumenverhältnis von Wasser zu Acetonitril kann dabei von 2 zu 98 bis 30 zu 70 Volumenanteilen reichen, wobei ein größerer Anteil Wasser zu verkürzten Retentionszeiten und verminderter Trennleistung führt.
Trifluoressigsäure im Anteil von 0,01 bis 0,5 Volumenteilen im Laufmittel sorgt für scharfe und symmetrische Peaks im Chromatogramm, während ein gleichzeitig vorhandener 10 bis 100 mal so hoher Anteil von Propionsäure im Laufmittel die Absenkung der lonisierungsausbeute im massenspektrometrischen Detektor durch Trifluoressigsäure wieder aufhebt. (SHOU ET AL: "Simple means to alleviate sensitivity loss by trifluoroacetic acid (TFA) mobile phases in the hydrophilic interaction chromatography-electrospray tandem mass spectrometric (HILIC-ESI/MS/MS) bioanalysis of basic compounds" JOURNAL OF CHROMATOGRAPHY B: BIOMEDICAL SCIENCES & APPLICATIONS, Bd. 825, Nr. 2, 5. 2005-02-05, Seiten 186-192) Die optimierten Volumenverhältnisse von Wasser / Acetonitril / Propionsäure / Trifluoressigsäure betragen 10 / 90 / 1 / 0,025. Unter diesen Bedingungen ergeben sich symmetrische und scharfe Peaks, die vollständig von Matrixbestandteilen abgetrennt sind (Figur 1).
Die Detektion der Analyten erfolgt nach positiver Ionisierung in einer Elektrospray-Ionenquelle mittels Tandem-Massenspektrometrie. Es werden die Fragmentionen 175,2 m/z → 70,1 m/z für Arginin, 181,2 m/z → 74,1 m/z für ¹³C₆-Arginin, 203,2 m/z → 172,1 m/z für SDMA, 203,2 m/z → 46,1 m/z fürADMA und 209,2 m/z → 70,1 m/z D₆-ADMA beobachtet. Diese Ionenspuren sind selektiv für die genannten Analyten und es kann auf diese Weise erstmalig auf die vollständige chromatographische Trennung von ADMA und SDMA verzichtetwerden.
Die Quantifizierung erfolgt über das Flächenverhältnis von Analyt zu internem Standard. FürArginin dient ¹³C₆-Arginin als interner Standard, für ADMA und SDMA dient D₆-ADMA als interner Standard.
Die Validierung des Verfahrens ergibt für alle beschriebenen biologischen Flüssigkeiten gute Ergebnisse. In der Tabelle I sind die einzelnen Daten zusammengefasst. Die Präzision in Serie wurde durch die 10-malige Bestimmung einer Probe innerhalb einer Arbeitsschicht bestimmt, die Wiederholpräzision durch die 6-malige Bestimmung einer Probe an verschiedenen Tagen.

**Tabelle 1**

| Substanz | Medium | Kalibrierbereich | Präzision in Serie | Wiederholpräzision |
|---|---|---|---|---|
| | | [µmol/l] | [%] | [%] |
| Arginin | Blutplasma | 0 - 150 | 4,48 | 4,66 |
| | Urin | 0 - 50 | 4,09 | 4,76 |
| | Zellkulturmedium | 0 - 50 | 4,59 | 2,92 |
| ADMA | Blutplasma | 0 - 3 | 5,52 | 7,67 |
| | Urin | 0 - 100 | 2,51 | 5,36 |
| | Zellkulturmedium | 0 - 3 | 3,40 | 7,68 |
| SDMA | Blutplasma | 0 - 4 | 3,93 | 4,86 |
| | Urin | 0 - 100 | 2,93 | 5,18 |
| | Zellkulturmedium | 0 - 4 | 10,8 | 6,48 |

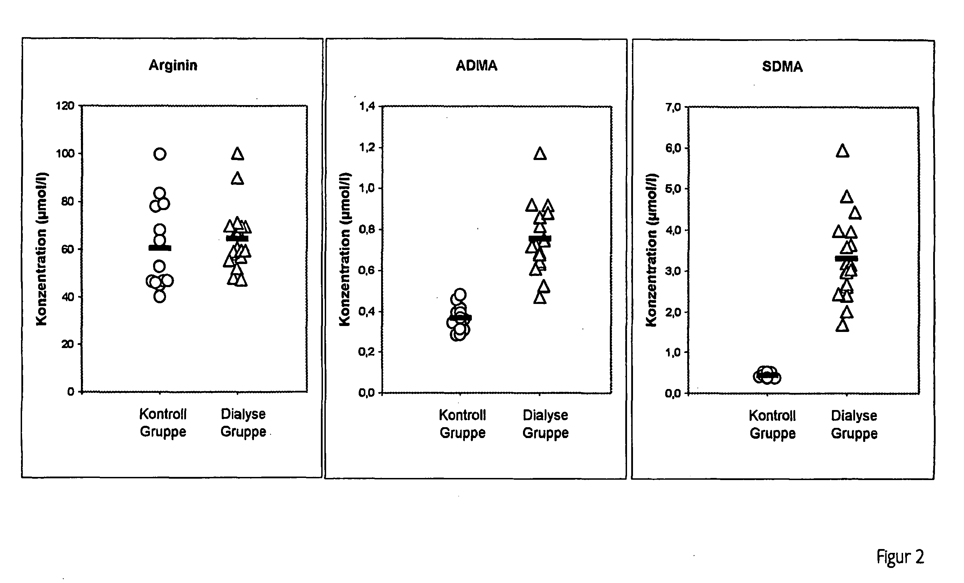

In der klinischen Forschung wurden Blutplasmawerte für Arginin, ADMA und SDMA von Normalprobanden und Dialysepatienten ermittelt (Figur 2). Wie man sieht, sind in der Dialysepatientengruppe die Werte für ADMA und SDMA hochsignifikant gegenüber der Kontrollgruppe erhöht, während für Arginin kein signifikanter Unterschied gefunden werden kann. Der Mittelwert für Arginin beträgt in der Kontrollgruppe 60,6 ± 18,27 µmol/l und in der Dialysegruppe 64,5 ± 13,41 µmol/l. Die entsprechenden Werte für ADMA sind 0,370 ± 0,061 µmol/l gegenüber 0,757 ± 0,165 µmol/l und für SDMA 0,449 ± 0,055 gegenüber 3,308 ± 1,048 µmol/l.

## Patentansprüche

1. Verfahren zur Bestimmung der Konzentration von asymmetrischem Dimethylarginin (ADMA) simultan mit Arginin und symmetrischem Dimethylarginin (SDMA) in biologischen Proben mittels HPLC MS-MS,
**dadurch gekennzeichnet,**
**dass** die Probenvorbereitung ausschließlich aus der Zugabe einer Lösung von isotopenmarkierten internen Standards, insbesondere ¹³C₆-Arginin und D₆-ADMA, und der Zugabe von 9 Teilen eines Gemisches aus Acetonitril/Propionsäure/ Trifluoressigsäure mit 99/1/0,025 Volumenanteilen auf einen Teil der Probe zur Fällung von hochmolekularen Eiweißstoffen besteht, ohne dass eine Derivatisierung oder Extraktion der Analyten erforderlich ist, die chromatographische Trennung der Analyten auf einer Silica-Normalphasen-HPLC-Säule unter Verwendung einer mobilen Phase aus Wasser/Acetonitril/Propionsäure/Trifluoressigsäure mit 10/90/1/0,025 Volumenanteilen erfolgt, und die Detektion und Quantifizierung mittels Tandem-Nassenspektrometrie erfolgt, wobei die Fragmentionen 175,2 m/z → 70,1 m/z für Arginin, 181,2 m/z → 74,1 m/z für ¹³C₆-Arginin, 203,2 m/z → 172,1 m/z für SDMA, 203,2 m/z → 46,1 m/z für ADMA und 209,2 m/z → 70,1 m/z D₆-ADMA beobachtet werden.

2. Verfahren nach Anspruch 1,
**dadurch gekennzeichnet,**
**dass** die Probenmatrix Blutplasma ist.

3. Verfahren nach Anspruch 1,
**dadurch gekennzeichnet,**
**dass** die Probenmatrix Urin ist.

4. Verfahren nach Anspruch 1,
**dadurch gekennzeichnet,**
**dass** die Proben matrix Zellkulturmedium ist.

## Claims

1. A method of determining the concentration of asymmetrical dimethylarginine (ADMA) simultaneously with arginine and symmetrical dimethylarginine (SDMA) in biological samples by means of HPLC-MS-MS, **characterized in that** sample preparation consists exclusively of the addition of a solution of isotope-labelled internal standards, in particular ¹³C₆-arginine and D₆-ADMA, and the addition of 9 parts of a mixture of 99:1:0.025 by volume acetonitrile/propionic acid/trifluoroacetic acid to one part of the sample to precipitate high molecular weight proteins without any need for derivatization or extraction of the analytes, the chromatographic separation of the analytes is effected on a silica normal phase HPLC column using a mobile phase of 10:90:1:0.025 by volume water/acetonitrile/propionic acid/trifluoroacetic acid, and the detection and quantification is effected by means of tandem mass spectrometry, observing the fractions 175.2 m/z → 70.1 m/z for arginine, 181.2 m/z → 74.1 m/z for ¹³C₆-arginine, 203.2 m/z → 172.1 m/z for SDMA, 203.2 m/z → 46.1 m/z for ADMA and 209.2 m/z → 70.1 m/z for D₆-ADMA.

2. A method according to claim 1, **characterized in that** the sample matrix is blood plasma.

3. A method according to claim 1, **characterized in that** the sample matrix is urine.

4. A method according to Claim 1, **characterized in that** the sample matrix is cell culture medium.

## Revendications

1. Procédé pour déterminer la concentration de diméthylarginine asymétrique (ADMA) simultanément à l'arginine et à la diméthylarginine symétrique (SDMA) dans des échantillons biologiques au moyen d'une CLHP-SM/SM,
**caractérisé en ce que** la préparation de l'échantillon se compose exclusivement de l'ajout d'une solution d'étalons internes standard marqués par des isotopes, en particulier à ¹³C₆-arginine et la D₆-ADMA, et de l'ajout de 9 fractions d'un mélange d'acétonitrile/acide propionique/acide trifluoroacétique avec 99/1/0,025 fractions en volume d'une partie de l'échantillon pour la précipitation des protéines de masses moléculaires élevées, sans qu'une dérivation ou une extraction des analytes soit nécessaire, **en ce que** la séparation chromatographique des analytes se fait sur une colonne de CLHP en phase normale à base de silice en utilisant une phase mobile composée d'eau/d'acétonitrile/d'acide propionique/d'acide trifluoroacétique à raison de 10/90/1/0,025 fractions en volume, et **en ce que** la détection et la quantification se font au moyen d'une spectrométrie de masse en tandem, les ions des fragments observés étant 175,2 m/z → 70,1 m/z pour l'arginine, 181,2 m/z → 74,1 m/z pour la ¹³C₆-arginine, 203,2 m/z → 172,1 m/z pour la SDMA, 203,2 m/z → 46,1 m/z pour l'ADMA et 209,2 m/z → 70,1 m/z pour la D₆-ADMA.

2. Procédé selon la revendication 1, **caractérisé en ce que** la matrice d'échantillon est du plasma sanguin.

3. Procédé selon la revendication 1, **caractérisé en ce que** la matrice d'échantillon est de l'urine.

4. Procédé selon la revendication 1, **caractérisé en ce que** la matrice d'échantillon est un milieu de culture cellulaire.
